# EUROPEAN PATENT APPLICATION

(11) **EP 3 323 825 A1**
(43) Date of publication of application: **23.05.2018**
(21) Application number: 16823808.7
(22) Date of filing: 05.07.2016
(51) Int. Cl.: C07K 14/00, A61K 38/16, A61P 37/04, A61P 35/00, A61P 31/00, G01N 33/58

(54) **POLYPEPTIDE COMPOUND AND PREPARATION METHOD AND USE THEREOF**

(30) Priority: 15.07.2015 CN 201510415514
(71) Applicant: Beijing Coislitong Industrial Co., Ltd., Chaoyang District, Beijing (CN); Han, Su, Chaoyang District, Beijing (CN)
(72) Inventor: HAN, Su, Chaoyang District, Beijing (CN)
(74) Representative: Hoffmann Eitle
(86) International application number: PCT/CN2016/088583
(87) International publication number: WO 2017/008661

(57) **Abstract**

The present invention discloses a polypeptide compound, a preparation method and an application thereof. The structural formula of the polypeptide compound is (X_{A}X_{B}X_{C}X_{D}X_{E}X_{F}X_{G}-X)₂KY or {(X_{A}X_{B}X_{C}X_{D}X_{E}X_{F}X_{G}-X)₂K}₂KY or {({X_{A}X_{B}X_{C}X_{D}X_{E}X_{E}X_{G}-X}₂K)₂K}₂KY where, X_{A}, X_{B}, X_{D}, X_{E} and X_{G} are one of aliphatic amino acid molecules respectively, X_{C} and X_{F} are aliphatic amino acid molecules or heterocyclic amino acid molecules, K is lysine (Lys, K), X or Y is null or any one or more amino acid or chemical groups. The polypeptide compound provided in the invention has an effect of enhancing the immune function of a body and has an application potential of being developed into a clinical medicine capable of enhancing the immune function of a body.

## Description

### Field of the Invention

The present invention relates to the bio-pharmaceutical field, in particular to a polypeptide compound molecule, a preparation method of the polypeptide compound, and an application of the polypeptide compound in preparation of medicines for enhancing immunity ability and vaccine immune response ability of an animal body.

### Background of the Invention

The genetic genes of organisms are stored in poly deoxynucleotide chains, and proteins that execute biological functions are coded in the genetic genes. Various proteins exist in organisms and they execute different biological functions to maintain vital activities. Though there are numerous kinds of proteins, they are essentially composed of 20 kinds of natural amino acids that exist in the natural world. Proteins differ significantly owing to the composition and sequence of amino acids. Generally speaking, molecules that contain 50 or more amino acids are referred to as proteins, peptide chains that contain 10 or more amino acids are referred to as polypeptides, and peptide chains that contain less than 10 amino acids are referred to as oligopeptides. The smallest functional small-peptide discovered up to now only contains 2 amino acids. Usually, functional small-peptides that are composed of 4 or more amino acids are commonly seen.

As the Human Genome Project has been finished and the Human Proteome Project has been developed, more and more functional protein segments will be discovered and applied as medicines in the bio-pharmaceutical field. A functional protein segment usually refers to a straight-chain polypeptide segment that is found as having a specific biological function. Such a functional protein segment usually is a peptide segment composed of two to tens of amino acids. The identified and discovered functional protein segments can be prepared in an artificial synthesis approach. Polypeptide medicines that have been developed and applied clinically include "oxytocin", "thymosin α1", and "thymopentin", etc. Polypeptide medicines available presently include "octreotide", which is prepared through artificial modification of natural peptide chains and used to treat hemorrhage of digestive tract and acromegaly, and "hirudin peptide", which has an anti-coagulation effect. The functional segments in proteins often can be screened to polypeptide segments that contain tens of amino acids or even as few as two amino acids. They set a basis for artificial synthesis and application of functional polypeptide segments.

In proteins, polypeptides or oligopeptides, the deletion, addition or substitution of a single amino acid, blocked of amino terminal (N terminal) or carboxyl terminal (C terminal), addition of any chemical group into the sequence or at the free end, etc., will result in changes of the original biological activity of the proteins, polypeptides, or oligopeptides. Designing, screening, and discovering new functional peptide fragments or seeking for efficient peptide fragments is an important link in the development of medicines.

### Contents of the Invention

To overcome the technical defects in the prior art, in a first aspect, the present invention provides a branched polypeptide compound, which has a structural formula expressed as (X_{A}X_{B}X_{C}X_{D}X_{E}X_{F}X_{G}-X)₂KY, or {(X_{A}X_{B}X_{C}X_{B}X_{E}X_{F}X_{G}-X)₂K}₂KY, or {({X_{A}X_{B}X_{C}X_{D}X_{E}X_{F}X_{G}-X}₂ K)₂K}₂KY;
X_{A}, X_{B}, X_{D}, X_{E} and X_{G} are one of aliphatic amino acid molecules respectively (may be the same or different), X_{C} and X_{F} are aliphatic amino acid molecules or heterocyclic amino acid molecules (may be the same or different), K is lysine (Lys, K), X and Y are null or any one or more amino acid or chemical groups.
the (X_{A}X_{B}X_{C}X_{D}X_{E}X_{F}X_{G}-X)₂KY structure is shown in formula 4: 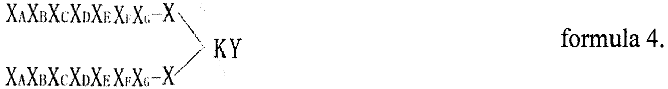 the {(X_{A}X_{B}X_{C}X_{D}X_{E}X_{F}X_{G}-X)₂K}₂KY structure is shown in formula 5: 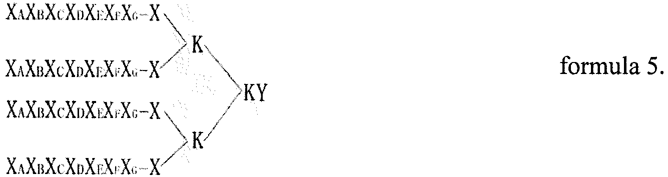 the {({X_{A}X_{B}X_{C}X_{D}X_{E}X_{F}X_{G}-X}₂K)₂K}₂KY structure is shown in formula 6:

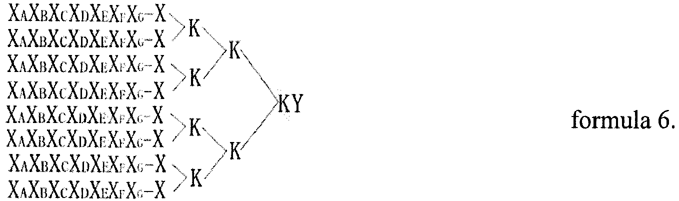

Where, X and Y are null, or any amino acid, or peptide fragments composed of any number of amino acids, or chemical groups that can connect amino acids or peptide fragments, and X and Y may be the same or different from each other; for example, X is null, and Y is glycine (Gly, G).

X_{A}, X_{B}, X_{D}, X_{E} and X_{G} are selected from alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E) or glutamine (Gln, Q) respectively, and X_{A}, X_{B}, X_{D}, X_{E} and X_{G} may be the same of different; X_{A} preferably is glycine (Gly, G), threonine (Thr, T), arginine (Arg, R), glutamate (Glu, E), alanine (Ala, A), lysine (Lys, K), leucine (Leu, L) or serine (Ser, S), more preferably is glycine (Gly, G), threonine (Thr, T) or arginine (Arg, R); X_{B} preferably is glutamine (Gln, Q), glutamate (Glu, E), arginine (Arg, R), alanine (Ala, A), threonine (Thr, T), leucine (Leu, L) or lysine (Lys, K), more preferably is glutamine (Gln, Q), arginine (Arg, R) or lysine (Lys, K); X_{D} preferably is arginine (Arg, R), serine (Ser, S), leucine (Leu, L), lysine (Lys, K), aspartate (Asp, D), glycine (Gly, G) or glutamate (Glu, E), more preferably is arginine (Arg, R), lysine (Lys, K) or glycine (Gly, G); X_{E} preferably is arginine (Arg, R), lysine (Lys, K), leucine (Leu, L) or glutamine (Gln, Q), more preferably is arginine (Arg, R) or lysine (Lys, K); X_{G} preferably is arginine (Arg, R), glycine (Gly, G), valine (Val, V), lysine (Lys, K), leucine (Leu, L) or glutamate (Glu, E), more preferably is arginine (Arg, R), glycine (Gly, G), valine (Val, V) or glutamate (Glu, E).

X_{C} and X_{F} are selected from alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E), glutamine (Gln, Q), tryptophan (Trp, W), histidine (His, H) or proline (Pro, P) respectively, and X_{C} and X_{F} may be the same or different; X_{C} preferably is lysine (Lys, K), proline (Pro, P), tryptophan (Trp, W), alanine (Ala, A), leucine (Leu, L), histidine (His, H) or aspartate (Asp, D), more preferably is proline (Pro, P), leucine (Leu, L) or histidine (His, H); X_{F} preferably is proline (Pro, P), glutamate (Glu, E), aspartate (Asp, D), histidine (His, H), glycine (Gly, G), alanine (Ala, A) or lysine (Lys, K), more preferably is proline (Pro, P), glutamate (Glu, E), aspartate (Asp, D), histidine (His, H) or lysine (Lys, K).

X preferably is tyrosine (Tyr, Y), arginine (Arg, R), serine (Ser, S), asparagine (Asn, N), glycine (Gly, G), glutamate (Glu, E) or null, more preferably is tyrosine (Tyr, Y), arginine (Arg, R) or null; Y preferably is glycine (Gly, G), alanine (Ala, A), cysteine (Cys, C) or null, more preferably is glycine (Gly, G), alanine (Ala, A) or cysteine (Cys, C).

The present invention further includes derivatives obtained through chemical modification or transformation on the basis of the side chain groups or terminal groups of the sequences of the amino acids in the polypeptide compound, such as:
A salt compound formed by the polypeptide compound with an organic acid or inorganic acid;
An ether, ester, glucoside, or glycoside compound, etc., which may be formed by the hydroxyl included in the polypeptide compound, but is not limited to compounds formed in such a way;
A thioether or thioglycoside compound, which may be formed by the sulfhydryl included in the polypeptide compound, or a compound containing disulfide bonds, which may be formed by the sulfhydryl included in the polypeptide compound with cysteine or peptide containing cysteine, but is not limited to compounds formed in such a way;
An acylate or alkylate compound, which may be formed by the amido included in the polypeptide compound, or a glucoside compound, etc., which may be formed by the amido included in the polypeptide compound with saccharides, but is not limited to compounds formed in such a way;
An ester or amide compound, etc., which may be formed by the carboxyl included in the polypeptide compound, but is not limited to compounds formed in such a way;
A glucoside, acylate, or alkylate compound, etc., which may be formed by the imino included in the polypeptide compound, but is not limited to compounds formed in such a way;
An ester, ether, glucoside, or glycoside compound, which may be formed by the phenolic hydroxyl included in the polypeptide compound, or a salt compound, which may be formed by the phenolic hydroxyl included in the polypeptide compound with organic alkalies or inorganic alkalies, but is not limited to compounds formed in such a way;
A coordinate, clathrate, or chelate compound formed by the polypeptide compound with metal ions;
A hydrate or solvent formed by the polypeptide compound.

In a second aspect, the present invention provides a pharmaceutical composition that contains the above-mentioned polypeptide compound, a geometrical isomer of the pharmaceutical composition, a pharmaceutically acceptable salt or solvated compound of the pharmaceutical composition, and the pharmaceutical composition in a form of pharmaceutical carrier or excipient.

In a third aspect, the present invention provides a method for preparing the above-mentioned polypeptide compound, in which a synthesis route of (X_{A}X_{B}X_{C}X_{D}X_{E}X_{F}X_{G}-X)₂KY is expressed by formula 1: 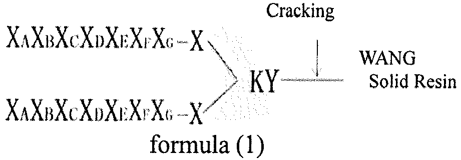
Y is fixed to WANG solid resin first, and then is bonded with lysine Fmoc-Lys(Fmoc)-OH (Lys, K) by condensation, to form a two-branch skeleton ">KY-WANG solid resin" with branch nodes;
Next, the two active amino terminals of K in the ">KY-WANG solid resin" are bonded with a X_{A}X_{B}X_{C}X_{D}X_{E}X_{F}X_{G}-X segment respectively, to form two-branch peptide (X_{A}X_{B}X_{C}X_{D}X_{E}X_{F}X_{G}-X)₂KY-WANG solid resin; or the two active amino terminals of K in the ">KY-WANG solid resin" are bonded with amino acids X, X_{G}, X_{F}, X_{E}, X_{D}, X_{C}, X_{B}, X_{A} by condensation in sequence, to obtain (X_{A}X_{B}X_{C}X_{D}X_{E}X_{F}X_{G}-X)₂KY-WANG solid resin;
Finally, the two-branch peptide is cracked from the WANG solid resin and then purified, to obtain a polypeptide compound (X_{A}X_{B}X_{C}X_{D}X_{E}X_{F}X_{G}-X)₂KY with two copies of X_{A}X_{B}X_{C}X_{D}X_{E}X_{F}X_{G}-X.

A synthesis route of {(X_{A}X_{B}X_{C}X_{D}X_{E}X_{F}X_{G}-X)₂K}₂KY structure is expressed by formula 2: 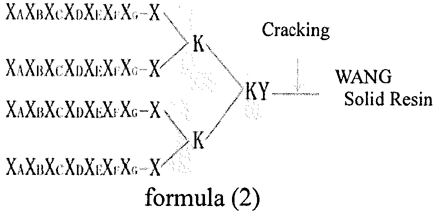
Y is fixed to WANG solid resin first, and then is bonded with lysine Fmoc-Lys(Fmoc)-OH by condensation, to form a two-branch skeleton ">KY-WANG solid resin" with branch nodes; then, the two active amino terminals of K in the ">KY-WANG solid resin" are bonded with the carboxyl terminals of lysine Fmoc-Lys(Fmoc)-OH by condensation, to form a four-branch skeleton ">K₂KY-WANG solid resin" with two branch nodes;
Next, the two active amino terminals of each lysine K in the ">K₂KY-WANG solid resin" are bonded with a X_{A}X_{B}X_{C}X_{D}X_{E}X_{F}X_{G}-X segment respectively, to form four-branch peptide (X_{A}X_{B}X_{C}X_{D}X_{E}X_{F}X_{G}-X)₂KY-WANG solid resin; or the two active amino terminals of K in the ">K₂KY-WANG solid resin" are bonded with amino acids X, X_{G}, X_{F}, X_{E}, X_{D}, X_{C}, X_{B}, X_{A} by condensation in sequence, to obtain (X_{A}X_{B}X_{C}X_{D}X_{E}X_{F}X_{G}-X)₄K₂KY-WANG solid resin;
Finally, the four-branch peptide is cleaved from the WANG solid resin and purified, to obtain a polypeptide compound (X_{A}X_{B}X_{C}X_{D}X_{E}X_{F}X_{G}-X)₂K}₂KY with four copies of X_{A}X_{B}X_{C}X_{D}X_{E}X_{F}X_{G}-X.

A synthesis route of {({X_{A}X_{B}X_{C}X_{D}X_{E}X_{F}X_{G}-X}₂K)₂K}₂KY structure is expressed by formula 3: 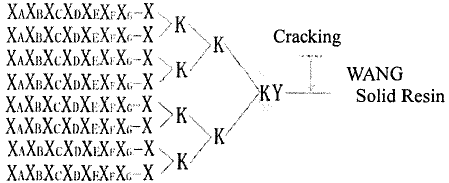 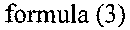
Y is fixed to WANG solid resin first, and then is bonded with lysine Fmoc-Lys(Fmoc)-OH by condensation, to form a two-branch skeleton ">KY-WANG solid resin" with branch nodes; the two active amino terminals of K are bonded with the carboxyl terminals of lysine Fmoc-Lys(Fmoc)-OH by condensation, to form a four-branch skeleton "K₂KY-WANG solid resin"; then, the two active amino terminals of K in the four-branch skeleton ">K₂KY-WANG solid resin" are bonded with the carboxyl terminals of lysine Fmoc-Lys(Fmoc)-OH by condensation, to form an eight-branch skeleton ">K₄K₂KY-WANG solid resin" with four branch nodes;
Next, the two active amino terminals of each lysine K in the ">K₄K₂KY-WANG solid resin" are bonded with a X_{A}X_{B}X_{C}X_{D}X_{E}X_{F}X_{G}-X segment respectively, to form eight-branch peptide (X_{A}X_{B}X_{C}X_{D}X_{E}X_{F}X_{G}-X)₈K₄K₂KY-WANG solid resin; or the two active amino terminals of K in the ">K₄K₂KY-WANG solid resin" are bonded with amino acids X, X_{G}, X_{F}, X_{E}, X_{D}, X_{C}, X_{B}, X_{A} by condensation in sequence, to obtain (X_{A}X_{B}X_{C}X_{D}X_{E}X_{F}X_{G}-X)₈K₄K₂KY-WANG solid resin;
Finally, the eight-branch peptide is cleaved from the WANG solid resin and purified, to obtain a polypeptide compound {({X_{A}X_{B}X_{C}X_{D}X_{E}X_{E}X_{G}-X}₂K)₂K}₂KY with eight copies of X_{A}X_{B}X_{C}X_{D}X_{E}X_{F}X_{G}-X.

Before the carboxyl terminal of K is condensed with Y-WANG solid resin, the two amido groups of K are protected, preferably with t-butyloxycarboryl (Boc) protection method and fluorenylmethoxycarbonyl (Fmoc) protection method;
Before the carboxyl terminals of the other two lysines are condensed with the two amido terminals of K in KY, the two amido groups of each lysine are protected; before the carboxyl terminal of the X_{A}X_{B}X_{C}X_{D}X_{E}X_{F}X_{G}-X is condensed with the amido terminal of each lysine, the amido group of the X_{A}X_{B}X_{C}X_{D}X_{E}X_{F}X_{G}-X is protected, preferably with t-butyloxycarboryl (Boc) protection method and fluorenylmethoxycarbonyl (Fmoc) protection method.

Specifically, the steps are as follows:
Step 1: protecting the two amido groups of the lysine K with an Fmoc protection method;
Step 2: fixing KY to the WANG solid resin with an automatic polypeptide synthesizer, in the following bonding sequence: KY-WANG solid resin;
   When the two-copy polypeptide compound is prepared, the two activated amino terminals of lysine in KY are further condensed with another two X_{A}X_{B}X_{C}X_{D}X_{E}X_{F}X_{G}-X fragments, to obtain the polypeptide compound (X_{A}X_{B}X_{C}X_{D}X_{E}X_{F}X_{G}-X)₂KY with two copies of X_{A}X_{B}X_{C}X_{D}X_{E}X_{F}X_{G}-X, which is fixed to the WANG solid resin; or the two activated amino terminals of the lysine in KY are further condensed with another two lysines K, in each of which the two amido groups have been protected with an Fmoc protection method, to obtain a two-branch skeleton K₂KY-WANG solid resin;
   When the four-copy polypeptide compound is prepared, the two activated amino terminals of each lysine in the two-branch skeleton "K₂" are further condensed with two X_{A}X_{B}X_{C}X_{D}X_{E}X_{F}X_{G}-X fragments, to obtain the polypeptide compound {(X_{A}X_{B}X_{C}X_{D}X_{E}X_{F}X_{G}-X)₂K}₂KY with four copies of X_{A}X_{B}X_{C}X_{D}X_{E}X_{F}X_{G}-X, which is fixed to the WANG solid resin; or the two activated amino terminals of each lysine in the two-branch skeleton "K₂" are further condensed with another two lysines K, in each of which the two amido groups have been protected with an Fmoc protection method, to obtain a four-branch skeleton K₄K₂KY-WANG solid resin;
   When the eight-copy polypeptide compound is prepared, the two activated amino terminals of each lysine in the four-branch skeleton "K₄" are further condensed with two X_{A}X_{B}X_{C}X_{D}X_{E}X_{F}X_{G}-X fragments, to obtain the polypeptide compound {({X_{A}X_{B}X_{C}X_{D}X_{E}X_{F}X_{G}-X}₂K)₂K)₂KY with eight copies of X_{A}X_{B}X_{C}X_{D}X_{E}X_{F}X_{G}-X, which is fixed to the WANG solid resin;
   Finally, the polypeptide compound is cleaved from the WANG solid resin with a TFA method, to obtain a crude polypeptide compound product;
   Step 3: purifying the crude polypeptide compound product with a chromatographic column (model: Daiso C18, 10µm, 100Å, 50x250mm), wherein, the mobile phase A is water solution that contains 0.05% trifluoroacetic acid and 2% acetonitrile, the mobile phase B is 90% acetonitrile/water, the flowing rate is 25mL/min., and the ultraviolet detection wavelength is 220nm; the eluting peak solution is collected and then freeze-dried, to obtain a white flocculent polypeptide compound.

In a fourth aspect, the present invention provides an application of the above-mentioned polypeptide compound in preparation of immunize medicines for humans or animals or medicines for enhancing the immune function of humans or animals.

In a fifth aspect, the present invention provides an application of a polypeptide compound prepared with the above-mentioned method in preparation of immunize medicines for humans or animals or medicines for enhancing the immune function of humans or animals.

In a sixth aspect, the present invention provides an application of the above-mentioned polypeptide compound or a polypeptide compound prepared with the above-mentioned method in preparation of medicines for inhibiting tumor growth in human or animal bodies.

The tumor is solid tumor (or residual tumor after medical operation) or hematological tumor (including leukaemia and lymphomata) in a human body.

The tumor includes but is not limited to sarcoma, liver cancer, colon cancer, lung cancer, stomach cancer, mammary cancer, and cervical cancer.

In a seventh aspect, the present invention provides an application of the above-mentioned polypeptide compound or a polypeptide compound prepared with the above-mentioned method in preparation of anti-infection or anti-virus medicines for humans or animals.

In an eighth aspect, the present invention provides an application of the above-mentioned polypeptide compound or a polypeptide compound prepared with the above-mentioned method in molecular tracers.

In a ninth aspect, the present invention provides an application of the above-mentioned polypeptide compound or a polypeptide compound prepared with the above-mentioned method in preparation of medicines for treating diseases of humans incurred by vascular proliferation (including, but not limited to application of medicines for treating maculopathy in fundus).

The polypeptide compound provided in the present invention bonds up multiple copies of peptide fragments composed of seven amino acid molecules each to form a branched polypeptide compound. The polypeptide compound is hopeful to be an effective constituent is a variety of medicines, and is applicable to preparation of medicines for preventing and curing a variety of diseases; especially, the polypeptide compound will be widely applied in preparation of immunity enhancing medicines; in addition, the polypeptide compound may also be used as a molecule tracer for inhibition of vascularization. In the case that the X_{C} and X_{F} in the X_{A}X_{B}X_{C}X_{D}X_{E}X_{F}X_{G} segment are heterocyclic amino acids, such as histidine (His), the resultant compound provides loci that can be labeled by iodine isotopes (I¹²⁵ or I¹³¹). Since the polypeptide compound is a branched peptide, it is highly resistant to aminopeptidase or carboxypeptidase in a physiological environment, and thereby the peptide molecules can carry markers more stably and be traced; thus, a risk that the peptide molecules are difficult to trace effectively owing to loss of markers is avoided better.

The polypeptide compound described in the present invention is insensitive to catabolic enzymes in a physiological environment, since it has a non-natural molecular structure. Therefore, the effective half-life of the polypeptide compound in organisms can be prolonged effectively, and thereby the biological effect of the polypeptide compound can last for longer time in bodies.

### Detailed Description of the Embodiments

The present invention relates to design and preparation of a branched polypeptide molecule (X_{A}X_{B}X_{C}X_{D}X_{E}X_{F}X_{G}-X)₂KY, or {(X_{A}X_{B}X_{C}X_{D}X_{E}X_{F}X_{G}-X)₂K}₂KY or {({X_{A}X_{B}X_{C}X_{D}X_{E}X_{F}X_{G}-X}₂K)₂K)₂KY, which contains multiple copies of X_{A}X_{B}X_{C}X_{D}X_{E}X_{F}X_{G}-X, where, X_{A}, X_{B}, X_{D}, X_{E} and X_{G} are aliphatic amino acid molecules, and are selected from one of alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E) and glutamine (Gln, Q) respectively, and may be the same of different; X_{C} and X_{F} may be aliphatic amino acid molecules or heterocyclic amino acid molecules (including tryptophan (Trp, W), histidine (His, H) and proline (Pro, P)), and are selected from one of alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E), glutamine (Gln, Q), tryptophan (Trp, W), histidine (His, H) and proline (Pro, P) respectively, and may be the same or different; K is lysine FmocLys-(Fmoc)-OH that contain two active amino groups, X and Y are null, or any amino acid, or peptide fragments composed by any number of amino acids, or chemical groups that can bond up amino acids and peptide fragments. By changing the kinds of X_{A}, X_{B}, X_{C}, X_{D}, X_{E}, X_{F}, and X_{G}, the polypeptide compound provided in the present invention can be adapted to treat a variety of diseases. Especially, the polypeptide compound provided in the present invention can enhance humoral immunity and cellular immunity ability of human or animal bodies. Thus, a medicine for enhancing immune function in clinical application (for humans or animals) can be developed from the polypeptide compound provided in the present invention.

In year 1963, an American scientist R. B. Merrifield invented a solid-phase synthesis method for extending a peptide chain by fixing the carboxyl terminal (C terminal) of amino acids in a target peptide to insoluble resin and controlling the amino terminal (N terminals) of amino acids bonded to the resin to have a condensation reaction with the carboxyl terminal of amino acids to be bonded; that is to say, the amino acids are condensed one by one starting from the carboxyl terminal (C terminal) of the polypeptide and extend continuously towards the amino terminal (N terminal) of the polypeptide segment. Therefore, when the condensation reaction of the amino acids is executed, the amido and side chain groups of the amino acids to be bonded must be protected to avoid reaction of them.

At present, commonly used protection methods include t-butyloxycarboryl (Boc) protection method and fluorenylmethoxycarbonyl (Fmoc) protection method. Therefore, whenever an amino acid has been bonded, a deprotection procedure must be executed (i.e., the amido on the solid-phase carrier is deprotected first, and then has a condensation reaction with the carboxyl of the next target amino acid to be bonded among amino acids in excessive quantity, to extend the peptide chain). The process is repeated through such steps, i.e., condensation, washing, deprotection, neutralization, washing, and then next cycle of condensation (for bonding the target amino acid) is executed, till required length of target peptide chain to be synthesized is reached. After the synthesis is finished, the target polypeptide is cracked from the resin with a TFA method, to obtain a crude product of target peptide.

The purification process is as follows:

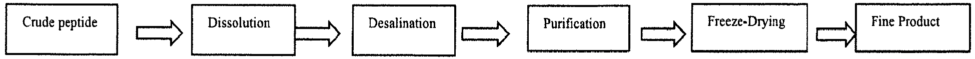

Usually, when linear-chain polypeptide is synthesized, lysine (Lys, K) Fmoc-Lys(Boc)-OH with one active amino is used, and the amido groups on the side chains are protected by BOC-OH to prevent them from participating in the condensation reaction. Therefore, only one amido group in the lysine (Lys, K) can have condensation reaction, and thereby the amino acids are bonded up one by one, and the peptide chain is extended linearly.

However, when the branched skeleton described in the present invention is synthesized, the branch point is a lysine Fmoc-Lys(Fmoc)-OH with two active amino groups, and the amido group on the side chain of the lysine also participates in the condensation reaction. Therefore, when the amino-acid condensation reaction proceeds from the lysine (Lys, K), branch chains will be developed, and a branched skeleton ">KY-WANG solid resin" will be formed. When the next cycle of condensation of lysine Fmoc-Lys(Fmoc)-OH is further executed on that basis, a four-branch skeleton ">K₂KY-WANG solid resin" will be formed; next, when the condensation of Fmoc-Lys(Fmoc) is continued further, an eight-branch skeleton ">K₄K₂KY-WANG solid resin" will be formed.

In the present invention, the lysine (Lys, K, Fmoc-Lys(Fmoc)-OH) with two active amino groups in KY is used as a branch point, and two amino acids for the next step of operation are bonded by condensation at the same time. If X_{A}X_{B}X_{C}X_{D}X_{E}X_{F}X_{G}-X is bonded, a two-branch peptide molecule (X_{A}X_{B}X_{C}X_{D}X_{E}X_{F}X_{G}-X)₂KY with two copies of X_{A}X_{B}X_{C}X_{D}X_{E}X_{F}X_{G}-X will be formed. Or, two K (Fmoc-Lys(Fmoc)-OH) may be bonded first to form a two-branch skeleton, and the two amino acids K for the next step of operation in "K₂" have two active amido groups respectively; thus, a branch point with four active amino groups is formed for condensation of amino acids (X_{A}X_{B}X_{C}X_{D}X_{E}X_{F}X_{G}-X) in the next step. Through such condensation in the sequence of X_{A}X_{B}X_{C}X_{D}X_{E}X_{F}X_{G}-X, the peptide chain is extended and a four-branch peptide molecule {(X_{A}X_{B}X_{C}X_{D}X_{E}X_{F}X_{G}-X)₂K}₂KY that contains four copies of X_{A}X_{B}X_{C}X_{D}X_{E}X_{F}X_{G}-X is formed. Alternatively, two K (Fmoc-Lys(Fmoc)-OH) may be bonded first to form a two-branch skeleton, and then four K (Fmoc-Lys(Fmoc)-OH) may be bonded to form a four-branch skeleton, and the four amino acids K for the next step of operation in "K₄" have two active amido groups respectively; thus, a branch point with eight active amino groups is formed for condensation of amino acids X_{A}X_{B}X_{C}X_{D}X_{E}X_{F}X_{G}-X) in the next step. Through such condensation in the sequence of X_{A}X_{B}X_{C}X_{D}X_{E}X_{F}X_{G}-X, the peptide chain is extended and an eight-branch peptide molecule {({X_{A}X_{B}X_{C}X_{D}X_{E}X_{F}X_{G}-X}₂K)₂K}₂KY that contains eight copies of X_{A}X_{B}X_{C}X_{D}X_{E}X_{E}X_{G}-X is formed. In that way, a multi-branch peptide molecule that contains sixteen copies, thirty-two copies, or more copies of X_{A}X_{B}X_{C}X_{D}X_{E}X_{F}X_{G}-X can be formed.

After the synthesis utilizing the WANG solid resin is finished, the peptide chain can be cracked from the WANG solid resin with a TFA method, so as to obtain a two-branch, four-branch, or eight-branch peptide molecule, or a peptide molecule with more branches, as described above.

Polypeptide synthesis is a conventional technique presently. Please see Chapter 3 "Chemical Synthesis and Purification of Polypeptides" in the book "Contemporary Theory and Application of Polypeptide Hormones" authored by Shuli Shen and published by Scientific and Technical Documentation Express (in 1998) for the principle and operation of synthesis and purification of polypeptides. The synthesis and preparation of the polypeptide compound in the present invention may be implemented with the above-mentioned solid phase synthesis method, but is not limited to that method.

Hereunder the present invention will be further detailed in embodiments, but those embodiments should not be understood as constituting any limitation to the present invention. Any modification or change made by those skilled in the art to the embodiments in the present invention according to the reveal in this document shall be deemed as falling in the scope of the present invention.

### Embodiment 1: synthesis of copy peptide fragment

The polypeptide compound in the present invention has the same copy peptide fragment X_{A}X_{B}X_{C}X_{D}X_{E}X_{F}X_{G}-X, regardless of whether it is in a two-branch, four-branch, or eight-branch structure. In the copy peptide fragment, X_{A}, X_{B}, X_{D}, X_{E} and X_{G} are aliphatic amino acid molecules (may be the same or different), and are selected from one or more of alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E) and glutamine (Gln, Q); X_{C} and X_{F} are aliphatic amino acid molecules or heterocyclic amino acid molecules (may be the same or different), and are selected from one or two of aliphatic amino acid molecule, tryptophan (Trp, W), histidine (His, H), and proline (Pro, P); X and Y may be null, or any one amino acid, or a peptide fragment composed of any number of amino acids, or a chemical group that can bond up amino acids and peptide fragments. The possible combinations are shown in Table 2-4, but the present invention is not limited to those combinations.

During the synthesis, an amino acid solid-phase synthesis method protected by organic chemical Fmoc protection may be used. The specific operation is as follows:
Step 1: a commercial raw material "X-WANG solid resin" or "X_{G}-WANG solid resin" is selected first, and the amido terminal of X or X_{G} is protected with an Fmoc protection method;
Step 2: X_{G} or X_{F} is selected to condense the amino acids one by one and extend the peptide chain, so as to synthesize an X_{A}X_{B}X_{C}X_{D}X_{E}X_{F}X_{G}-X copy peptide fragment:
   In this embodiment, the synthesis of the copy peptide fragment is implemented by condensing the amino acids one by one from the carboxyl terminal (C) to the amino terminal (N) of the polypeptide and thereby extending the chain on an automatic polypeptide synthesizer (model ABI433A), and then cracking the target polypeptide from the WANG solid resin with a TFA method after the synthesis is finished.
   On the automatic polypeptide synthesizer (model ABI433A), X or X_{G} is fixed to the WANG solid resin (the WANG solid resin is a carrier for Fmoc protection in the solid phase peptide synthesis) first, and then amino acids (X_{G}, X_{F}, X_{E}, X_{D}, X_{C}, X_{B}, X_{A} or X_{F}, X_{E}, X_{D}, X_{C}, X_{B}, X_{A}) are bonded by condensation. The actual bonding sequence is X_{A}X_{B}X_{C}X_{D}X_{E}X_{F}X_{G}-X-WANG solid resin. Then, the fragment is cracked from the WANG solid resin. Thus, a crude copy peptide fragment X_{A}X_{B}X_{C}X_{D}X_{E}X_{F}X_{G}-X is obtained.
Step 3: purification of X_{A}X_{B}X_{C}X_{D}X_{E}X_{F}X_{G}-X copy peptide fragment

The crude product is purified with a chromatographic column (model: Daiso C18, 10µm, 100Å, 50x250mm), wherein, the mobile phase A in the chromatographic operation is water solution that contains 0.05% trifluoroacetic acid and 2% acetonitrile, the mobile phase B is 90% acetonitrile/water, the flowing rate is 25mL/min., and the ultraviolet detection wavelength is 220nm. The eluting peak solution is collected and then freeze-dried. Thus, white flocculent X_{A}X_{B}X_{C}X_{D}X_{E}X_{F}X_{G}-X copy peptide fragments are obtained. Then, the copy peptide fragments are packed in a sealed state and stored in a refrigerator for later use; the purity of the copy peptide fragments may be >99%.

X_{A}, X_{B}, X_{C}, X_{D} and X_{E}, X_{F}, and X_{G} are commercially available. When the polypeptide compound in the present invention is prepared, X-solid resin or X_{G}-solid resin purchased commercially may be also used as a raw material, and amino acids are further condensed to the amino terminal of X or X_{G}, so as to obtain the copy peptide fragment described in the present invention. Some copy peptide fragments obtained through synthesis and their molecular weights measured by mass spectrometer measurement are listed in Table 1.

**Table 1. List of Groups in the Copy Peptide Fragment X_{A}X_{B}X_{C}X_{D}X_{E}X_{F}X_{G}-X**

| Embodiment | X_{A} | X_{B} | X_{C} | X_{D} | X_{E} | X_{F} | X_{G} | X | Molecular Weight | |
|---|---|---|---|---|---|---|---|---|---|---|
| | | | | | | | | | Theoretical | Actual |
| 1-1 | G | Q | P | R | R | P | R | R | 1022.17 | 1023.12 |
| 1-2 | T | E | P | R | K | E | G | Null | 815.87 | 816.48 |
| 1-3 | R | R | P | R | K | D | V | Y | 1089.25 | 1090.03 |
| 1-4 | T | R | P | R | R | H | G | Null | 878.98 | 879.65 |
| 1-5 | E | A | K | S | Q | G | G | SN | 876.87 | 877.57 |
| 1-6 | A | T | W | L | R | P | R | R | 1055.24 | 1056.28 |
| 1-7 | K | L | A | K | L | A | K | Null | 771.0 | 771.68 |
| 1-8 | L | K | A | D | K | A | K | G | 829.98 | 830.69 |

It is seen from the result in the Table 1: the deviation of the measured molecular weight of the copy peptide fragment synthesized in the present invention from the theoretical molecular weight is smaller than 1%, which proves that the copy peptide fragment is right the copy peptide fragment in the corresponding embodiment.

This embodiment part is provided to disclose the content of the copy peptide fragment, rather than limit the present invention. The actual synthesis may be executed according to the description in the following embodiments.

A branched skeleton is prepared with the method described in the present invention, wherein, the amino acid at the branch node is Fmoc-Lys(Fmoc)-OH that carries two active amido groups, which provide branch loci for the subsequent amino-acid condensation and bonding reactions. Therefore, a two-branch skeleton ">KY-WANG solid resin", four-branch skeleton ">K₂KY-WANG solid resin", or eight-branch skeleton ">K₄K₂KY-WANG solid resin", ..., can be synthesized. Then, amino acids in which the amino groups are protected are selected according to the conventional peptide extension reaction, and peptide extension from the branch nodes is executed, so as to prepare the two-branch peptide molecule (X_{A}X_{B}X_{C}X_{D}X_{E}X_{F}X_{G}-X)₂KY, four-branch peptide molecule {(X_{A}X_{B}X_{C}X_{D}X_{E}X_{F}X_{G}-X)₂K}₂KY, or eight-branch peptide molecule {({X_{A}X_{B}X_{C}X_{D}X_{E}X_{F}X_{G}-X}₂K)₂K}₂KY in the present invention. In view that the peptide chain extension process is a mature technique, the details of the synthesis steps will not be described any more here.

### Embodiment 2: Synthesis of two-branch peptide (X_{A}X_{B}X_{C}X_{D}X_{E}X_{F}X_{G}-X)₂KY

The structure and synthesis route of the polypeptide compound (X_{A}X_{B}X_{C}X_{D}X_{E}X_{F}X_{G}-X)₂KY that contains two copies of X_{A}X_{B}X_{C}X_{D}X_{E}X_{F}X_{G}-X provided in the present invention are represented by formula 1:

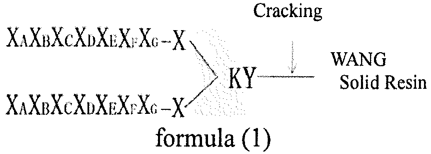

Where, X_{A}, X_{B}, X_{C}, X_{D}, X_{E}, X_{F} and X_{G} are selected from any one of alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E) and glutamine (Gln, Q) respectively, and may be the same of different; wherein, alternatively, X_{C} and X_{F} may be selected from heterocyclic amino acids, and may be any of tryptophan (Trp, W), histidine (His, H) and proline (Pro, P) respectively; K is lysine Fmoc-Lys(Fmoc)-OH that contains two active amino groups; X or Y may be null, or any one amino acid, or a segment composed of a plurality of amino acids, or a chemical group that has an amino acid bonding function;
In this embodiment, an amino acid solid-phase synthesis method protected by organic chemical Fmoc protection is used. The specific operation is as follows:
Step 1: the amido groups of the lysine are protected with an Fmoc protection method;
Step 2: synthesis of polypeptide compound (X_{A}X_{B}X_{C}X_{D}X_{E}X_{F}X_{G}-X)₂KY:
   In this embodiment, the synthesis of the polypeptide is implemented by condensing the amino acids one by one from the carboxyl terminal (C) to the amino terminal (N) of the polypeptide and thereby extending the chain on an automatic polypeptide synthesizer (model ABI433A), and then cracking the target polypeptide from the WANG solid resin with a TFA method after the synthesis is finished.
   On the automatic polypeptide synthesizer (model ABI433A), Y is fixed to the WANG solid resin (the WANG solid resin is a carrier for Fmoc protection in the solid phase peptide synthesis) first, and then lysine (Fmoc-Lys(Fmoc)-OH) is bonded by condensation. The actual bonding sequence is Lys-Y-WANG solid resin. Thus, a two-branch skeleton ">KY-WANG solid resin" with branch nodes is formed; since the terminal lysine has two activated amido groups, the two active amino terminals of K in the ">KY-WANG solid resin" will react with another two X_{A}X_{B}X_{C}X_{D}X_{E}X_{F}X_{G}-X segments. Thus, extended two-branch peptide (X_{A}X_{B}X_{C}X_{D}X_{E}X_{F}X_{G}-X)₂KY-WANG solid resin is obtained, i.e., a polypeptide compound (X_{A}X_{B}X_{C}X_{D}X_{E}X_{F}X_{G}-X)₂KY-WANG solid resin with two copies of X_{A}X_{B}X_{C}X_{D}X_{E}X_{F}X_{G}-X, which is fixed to the WANG solid resin, is obtained.
   In this step, X_{A}X_{B}X_{C}X_{D}X_{E}X_{F}X_{G}-X segments may be synthesized as described in the embodiment 1 first, and then they may be condensed with the Lys-Y-WANG solid resin; or, the two active amino terminals of K in the ">KY-WANG solid resin" may be bonded with X, X_{G}, X_{F}, X_{E}, X_{D}, X_{C}, X_{B} and X_{A} in sequence. Finally, the target polypeptide compound may be cracked from the WANG solid resin with a TFA method, to obtain a crude product of (X_{A}X_{B}X_{C}X_{D}X_{E}X_{F}X_{G}-X)₂KY polypeptide compound.
Step 3: purification of (X_{A}X_{B}X_{C}X_{D}X_{E}X_{F}X_{G}-X)₂KY polypeptide compound

The crude product is purified with a chromatographic column (model: Daiso C18, 10µm, 100Å, 50x250mm), wherein, the mobile phase A in the chromatographic operation is water solution that contains 0.05% trifluoroacetic acid and 2% acetonitrile, the mobile phase B is 90% acetonitrile/water, the flowing rate is 25mL/min., and the ultraviolet detection wavelength is 220nm. The eluting peak solution is collected and then freeze-dried. Thus, a white flocculent (X_{A}X_{B}X_{C}X_{D}X_{E}X_{E}X_{G}-X)₂KY polypeptide compound is obtained. Then, the polypeptide compound is packed in a sealed state and stored in a refrigerator for later use; the purity of the polypeptide compound may be >99%.

X_{A}, X_{B}, X_{C}, X_{D}, X_{E}, X_{F} and X_{G} are commercially available. When the polypeptide compound in the present invention is prepared, WANG solid resin-Y purchased commercially may also be used as a raw material, and amino acids may be condensed to the terminals of Y with the above-mentioned method, so as to obtain the polypeptide compound in the present invention.

The selection of segments for the series of two-branch peptides (X_{A}X_{B}X_{C}X_{D}X_{E}X_{F}X_{G}-X)₂KY synthesized with the method taught in this embodiment is shown in Fig. 2.

**Table 2. List of Segments of Two-Branch Peptide (X_{A}X_{B}X_{C}X_{D}X_{E}X_{F}X_{G}-X)₂KY**

| Embodiment | X_{A} | X_{B} | X_{C} | X_{D} | X_{E} | X_{F} | X_{G} | X | Y | Embodiment | X_{A} | X_{B} | X_{C} | X_{D} | X_{E} | X_{F} | X_{G} | X | Y |
|---|---|---|---|---|---|---|---|---|---|---|---|---|---|---|---|---|---|---|---|
| 2-1 | G | Q | P | R | R | P | R | R | G | 2-9 | T | K | L | K | K | H | G | Null | C |
| 2-2 | T | E | P | R | K | E | G | Null | A | 2-10 | R | R | H | G | R | H | G | Null | G |
| 2-3 | R | R | P | R | K | D | V | Y | A | 2-11 | K | E | K | E | R | H | G | Null | Null |
| 2-4 | T | R | P | R | R | H | G | Null | G | 2-12 | T | K | D | L | K | E | K | Null | G |
| 2-5 | E | A | K | S | Q | G | G | SN | Null | 2-13 | R | L | P | R | R | P | L | Null | C |
| 2-6 | A | T | W | L | R | P | R | R | G | 2-14 | T | K | L | R | R | K | E | Null | G |
| 2-7 | K | L | A | K | L | A | K | Null | C | 2-15 | S | R | P | R | R | G | G | E | Null |
| 2-8 | L | K | A | D | K | A | K | G | A | 2-16 | G | Q | P | R | K | E | V | Y | G |

### Embodiment 3: Synthesis of four-branch peptide {(X_{A}X_{B}X_{C}X_{D}X_{E}X_{F}X_{G}-X)₂K}₂KY

The structure and synthesis route of the polypeptide compound {(X_{A}X_{B}X_{C}X_{D}X_{E}X_{F}X_{G}-X)₂K}₂KY that contains four copies of X_{A}X_{B}X_{C}X_{D}X_{E}X_{F}X_{G}-X provided in the present invention are represented by formula 2. The definitions of the segments are the same as those in the embodiment 2:

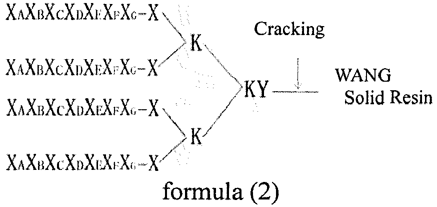

In this embodiment, an amino acid solid-phase synthesis method protected by organic chemical Fmoc protection is used. The specific operation is as follows:
Step 1: the amido groups of the lysine are protected with an Fmoc protection method;
Step 2: synthesis of polypeptide compound {(X_{A}X_{B}X_{C}X_{D}X_{E}X_{F}X_{G}-X)₂K}₂KY:
   In this embodiment, the synthesis of the polypeptide is implemented by condensing the amino acids one by one from the carboxyl terminal (C) to the amino terminal (N) of the polypeptide and thereby extending the chain on an automatic polypeptide synthesizer (model ABI433A), and then cracking the target polypeptide from the WANG solid resin with a TFA method after the synthesis is finished.
   On the automatic polypeptide synthesizer (model ABI433A), Y is fixed to the WANG solid resin (the WANG solid resin is a carrier for Fmoc protection in the solid phase peptide synthesis) first, and then lysine (Fmoc-Lys(Fmoc)-OH) is bonded by condensation. The actual bonding sequence is Lys-Y-WANG solid resin. Thus, a two-branch skeleton ">KY-WANG solid resin" with branch nodes is formed; since the terminal lysine has two activated amido groups, the two activated amido groups will have a condensation reaction with the carboxyl terminals of another two lysines (K, here, the two amido groups are protected). Thus, two extended branch skeletons K₂KY-WANG solid resin are obtained. Here, the amino terminals of the lysine in KY are bonded with two lysines (K), each of which has two active amido groups; thus, a four-branch skeleton ">K₂KY-WANG solid resin" with branch nodes is formed; the condensation with two X_{A}X_{B}X_{C}X_{D}X_{E}X_{F}X_{G}-X segments is executed further on each lysine (K) that has two active amino groups in "K₂"; thus, a polypeptide compound {(X_{A}X_{B}X_{C}X_{D}X_{E}X_{F}X_{G}-X)₂K}₂KY-WANG solid resin with four copies of X_{A}X_{B}X_{C}X_{D}X_{E}X_{F}X_{G}-X, which is fixed to the WANG solid resin, is obtained.
   In this step, X_{A}X_{B}X_{C}X_{D}X_{E}X_{F}X_{G}-X segments may be synthesized as described in the embodiment 1 first, and then they may be condensed with the K₂KY-WANG solid resin; or, the two active amino terminals of K in the ">K₂KY-WANG solid resin" may be bonded with X, X_{G}, X_{F}, X_{E}, X_{D}, X_{C}, X_{B} and X_{A} in sequence. Finally, the target polypeptide compound may be cracked from the WANG solid resin with a TFA method, to obtain a crude product of {(X_{A}X_{B}X_{C}X_{D}X_{E}X_{F}X_{G}-X)₂K}₂KY polypeptide compound.
Step 3: purification of {(X_{A}X_{B}X_{C}X_{D}X_{E}X_{F}X_{G}-X)₂K}₂KY polypeptide compound

The purification is the same as that in the embodiment 2. Finally, a white flocculent {(X_{A}X_{B}X_{C}X_{D}X_{E}X_{F}X_{G}-X)₂K}₂KY polypeptide compound is obtained. Then, the polypeptide compound is packed in a sealed state and stored in a refrigerator for later use; the purity of the polypeptide compound may be >99%.

A series of four-branch peptides {(X_{A}X_{B}X_{C}X_{D}X_{E}X_{F}X_{G}-X)₂K}₂KY are obtained with the method described in this embodiment. Please see Table 3 for the selection of the groups.

**Table 3. List of Segments of Four-Branch Peptide {(X_{A}X_{B}X_{C}X_{D}X_{E}X_{F}X_{G}-X)₂K}₂KY**

| Embodiment | X_{A} | X_{B} | X_{C} | X_{D} | X_{E} | X_{F} | X_{G} | X | Y | Embodiment | X_{A} | X_{B} | X_{C} | X_{D} | X_{E} | X_{F} | X_{G} | X | Y |
|---|---|---|---|---|---|---|---|---|---|---|---|---|---|---|---|---|---|---|---|
| 4-1 | G | Q | P | R | R | P | R | R | G | 4-9 | T | K | L | K | K | H | G | Null | C |
| 4-2 | T | E | P | R | K | E | G | Null | A | 4-10 | R | R | H | G | R | H | G | Null | G |
| 4-3 | R | R | P | R | K | D | V | Y | A | 4-11 | K | E | K | E | R | H | G | Null | Null |
| 4-4 | T | R | P | R | R | H | G | Null | G | 4-12 | T | K | D | L | K | E | K | Null | G |
| 4-5 | E | A | K | S | Q | G | G | SN | Null | 4-13 | R | L | P | R | R | P | L | Null | C |
| 4-6 | A | T | W | L | R | P | R | R | G | 4-14 | T | K | L | R | R | K | E | Null | G |
| 4-7 | K | L | A | K | L | A | K | Null | C | 4-15 | S | R | P | R | R | G | G | E | Null |
| 4-8 | L | K | A | D | K | A | K | G | A | 4-16 | G | Q | P | R | K | E | V | Y | G |

### Embodiment 4: Synthesis of eight-branch peptide {({X_{A}X_{B}X_{C}X_{D}X_{E}X_{F}X_{G}-X}₂K)₂K}₂KY

The structure and synthesis route of the polypeptide compound {({X_{A}X_{B}X_{C}X_{D}X_{E}X_{E}X_{G}-X}₂K)₂K}₂KY that contains eight copies of X_{A}X_{B}X_{C}X_{D}X_{E}X_{F}X_{G}-X provided in the present invention are represented by formula 3. The definitions of the segments are the same as those in the embodiment 2:

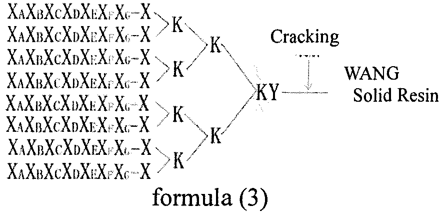

In this embodiment, an amino acid solid-phase synthesis method protected by organic chemical Fmoc protection is used. The specific operation is as follows:
Step 1: the amido groups of the lysine are protected with an Fmoc protection method;
Step 2: synthesis of polypeptide compound {({X_{A}X_{B}X_{C}X_{D}X_{E}X_{F}X_{G}-X)₂K)₂K}₂KY:
   In this embodiment, the synthesis of the polypeptide is implemented by condensing the amino acids one by one from the carboxyl terminal (C) to the amino terminal (N) of the polypeptide and thereby extending the chain on an automatic polypeptide synthesizer (model ABI433A), and then cracking the target polypeptide from the WANG solid resin with a TFA method after the synthesis is finished.
   On the automatic polypeptide synthesizer (model ABI433A), Y is fixed to the WANG solid resin (the WANG solid resin is a carrier for Fmoc protection in the solid phase peptide synthesis) first, and then lysine (Lys, K) is bonded by condensation. The actual bonding sequence is Lys-Y-WANG. Thus, a two-branch skeleton ">KY-WANG solid resin" with branch nodes is formed; since the terminal lysine has two activated amido groups, the two activated amido groups will have a condensation reaction with the carboxyl terminals of another two lysines (K, here, the two amido groups are protected). Thus, two extended branch skeletons K₂KY-WANG solid resin are obtained. Here, the amino terminals of the lysine in KY are bonded with two lysines (K, here, the two amido groups are protected), each of which has two active amido groups; thus, a four-branch skeleton ">K₂KY-WANG solid resin" with branch nodes is formed; the terminals of two lysines that have a single activated amido group each in "K₂" have a condensation reaction with the carboxyl terminals of another two lysines (K, here, the two amido groups are protected). Thus, extended four-branch skeletons K₄K₂KY-WANG solid resin are obtained. Here, the amino terminals of the terminal lysine in K₂KY are bonded with four lysines (K), each of which has two active amido groups; thus, an eight-branch skeleton ">K₄K₂KY-WANG solid resin" with branch nodes is formed; the condensation with two X_{A}X_{B}X_{C}X_{D}X_{E}X_{F}X_{G}-X segments is executed further on each lysine (K) that has two active amino groups in "K₄" in the "K₄K₂KY-WANG solid resin"; thus, a polypeptide compound {({X_{A}X_{B}X_{C}X_{D}X_{E}X_{F}X_{G}-X)₂K)₂K}₂KY-WANG solid resin with eight copies of X_{A}X_{B}X_{C}X_{D}X_{E}X_{F}X_{G}-X, which is fixed to the WANG solid resin, is obtained.
   In this step, X_{A}X_{B}X_{C}X_{D}X_{E}X_{F}X_{G}-X segments may be synthesized first, and then they may be condensed with the {(K)₂K}₂KY-WANG solid resin; or, the two active amino terminals of K in the ">K₄K₂KY-WANG solid resin" may be bonded with X, X_{G}, X_{F}, X_{E}, X_{D}, X_{C}, X_{B} and X_{A} in sequence. Finally, the target polypeptide compound may be cracked from the WANG solid resin with a TFA method, to obtain a crude product of {({X_{A}X_{B}X_{c}X_{D}X_{E}XᵣX_{G}-X}₂K)₂K}₂KY polypeptide compound.
Step 3: purification of polypeptide compound {({X_{A}X_{B}X_{C}X_{D}X_{E}X_{F}X_{G}-X)₂K)₂K)₂KY:
   The purification is the same as that in the embodiment 2. Finally, a white flocculent {({X_{A}X_{B}X_{C}X_{D}X_{E}X_{F}X_{G}-X}₂K)₂K)₂KY polypeptide compound is obtained. Then, the polypeptide compound is packed in a sealed state and stored in a refrigerator for later use; the purity of the polypeptide compound may be >99%.

A series of eight-branch peptides {({X_{A}X_{B}X_{C}X_{D}X_{E}X_{F}X_{G}-X}₂K)₂K}₂KY are obtained with the method described in this embodiment. Please see Table 4 for the selection of the segments.

**Table 4. List of Segments of Eight-Branch Peptide {({X_{A}X_{B}X_{C}X_{D}X_{E}X_{F}X_{G}-X}₂K)₂K}₂KY**

| Embodiment | X_{A} | X_{B} | X_{C} | X_{D} | X_{E} | X_{F} | X_{G} | X | Y | Embodiment | X_{A} | X_{B} | X_{C} | X_{D} | X_{E} | X_{F} | X_{G} | X | Y |
|---|---|---|---|---|---|---|---|---|---|---|---|---|---|---|---|---|---|---|---|
| 8-1 | G | Q | P | R | R | P | R | R | G | 8-9 | T | K | L | K | K | H | G | Null | C |
| 8-2 | T | E | P | R | K | E | G | Null | A | 8-10 | R | R | H | G | R | H | G | Null | G |
| 8-3 | R | R | P | R | K | D | V | Y | A | 8-11 | K | E | K | E | R | H | G | Null | Null |
| 8-4 | T | R | P | R | R | H | G | Null | G | 8-12 | T | K | D | L | K | E | K | Null | G |
| 8-5 | E | A | K | S | Q | G | G | SN | Null | 8-13 | R | L | P | R | R | P | L | Null | C |
| 8-6 | A | T | W | L | R | P | R | R | G | 8-14 | T | K | L | R | R | K | E | Null | G |
| 8-7 | K | L | A | K | L | A | K | Null | C | 8-15 | S | R | P | R | R | G | G | E | Null |
| 8-8 | L | K | A | D | K | A | K | G | A | 8-16 | G | Q | P | R | K | E | V | Y | G |

Since the polypeptide compound in the present invention is a sort of organic molecules with biological activity, their biological effects depend on their amino acid sequence and structure. Any change of a single amino acid in the protein or peptide sequence may result in changes of the biological activity. Hereunder the biological activity and efficacy of the polypeptide compound provided in the present invention will be described in specific experimental examples.

### Experimental example 1: Experiment of immune effect of the polypeptide compound provided in the present invention among birds and poultries (chicks)

Newcastle Disease Virus (NDV) can cause a hemagglutination phenomenon among chickens, which is a specific antibody neutralization reaction. The principle is that the hemagglutinin produced by the virus can cause agglutination of red blood cells. However, if a specific antibody is used to counteract the virus first before the virus is added into red blood cells, the hemagglutination phenomenon will not occur any more. Such a test is referred to as a hemagglutination inhibition test (HI), and the maximum multiple of dilution of the anti-serum used in the detection is the titer of the antibody. The higher the titer of the tested antibody is, the better the immune effect is.

The HI method has the following advantages:
1. High sensitivity: the HI method can detect antibody in a trace quantity, and the result is relatively accurate, the reaction is one of sensitive serologic detection reactions;
2. High specificity: the virus that causes agglutination of red blood cells can only be inhibited by a specific antibody;
3. High detection speed: only about 2h is required in a HI test to judge the result;
4. The HI test doesn't have any high requirement for the environment, and the operation is simple and quick, a large quantity of samples can be detected in one test.

The polypeptide compounds obtained in the embodiments 2-1∼2-16, 4-1∼4-16, and 8-1∼8-16 in the present invention are used for test of antibody titer among chicks: Live NDV vaccine (CS2 strain, from Chengdu Tianbond Biological Products Co., Ltd.) and the polypeptide compound provided in the present invention are inoculated to SPF chicks, and then the HI antibody formation effect of the polypeptide compound against live NDV vaccine in the bodies of SPF chicks is tested, so as to ascertain the immune effect of the polypeptide compound provided in the present invention against live NDV vaccine (antigen).

The experimental method is as follows: 7-day-old specific pathogen free chicks (abbreviated as SPF chicks) are chosen. The SPF chicks are divided into 8 groups, with 12 chicks in each group. Subcutaneous vaccination is carried out in the axillary region of a wing of each SPF chick in the following groups. The SPF chicks in each group are bred in isolators. About 1ml venous blood is taken under a wing of each SPF chick on the fourteenth day after inoculation, the serum is separated, and HI detection is carried out. The detection results of the embodiments 2-1, 2-3, 4-9, 4-10, 8-14 and 8-16 (corresponding to the experimental groups 1∼6 sequentially) are shown in Table 5 (only a part of the detection results of the polypeptide compounds are listed). The results of the other embodiments have little difference with those shown in Table 5, and are omitted here. See the "Experiment Course of Animal Immunology" authored by Xin Guo and published by the Press of China Agricultural University in 2007 for the details of operation.
Blank group: 0.3ml normal saline is injected;
Reference group: 0.3ml live NDV vaccine (abbreviated as "vaccine", CS2 strain) is inoculated;
Experimental group: 0.3ml vaccine mixed with 02µg polypeptide compound provided in the present invention is inoculated.

**Table 5. Result of Immunity Experiment of SPF Chicks**

| Group | Inoculated Substance | | Average Antibody Titer |
|---|---|---|---|
| | Vaccine | Embodiment | |
| Blank group | No | No | Negative |
| Reference group | Vaccine | No | 8.2log4 |
| Experimental group 1 | Vaccine | 2-1 | 9.2log5 |
| Experimental group 2 | Vaccine | 2-3 | 9.3log4 |
| Experimental group 3 | Vaccine | 4-9 | 9.6log4 |
| Experimental group 4 | Vaccine | 4-10 | 9.7log3 |
| Experimental group 5 | Vaccine | 8-14 | 10.7log3 |
| Experimental group 6 | Vaccine | 8-16 | 10.6log4 |

| | | | |
|---|---|---|---|
| Note: "Negative" refers to that the HI antibody titer is zero; | | | |

The dietetic activities of the SPF chicks in the groups are normal during the experiment, no adverse reaction is seen, and no SPF chick dies. That indicates the polypeptide compound provided in the present invention is safe to use. The results in Table 5 indicate that the average HI antibody titer (experimental groups 1-8) is higher after the polypeptide compound provided in the present invention is added, when compared with the blank group and reference group (vaccine is inoculated solely). Thus, it is proved that a good immunity enhancement effect can be attained when the polypeptide compound provided in the present invention is used in combination with the vaccine, and the average HI antibody titer is higher than that of the reference by 1 or more.

Experimental example 2: Experiment on the influence of different branches of the polypeptide compound provided in the present invention on the immune effect under a condition of the same multi-copy group

An experiment on the immune effect of two groups of polypeptide compounds (groups I and II) in the present invention is carried out with the method described in the experimental example 1. In the two groups of polypeptide compounds, the multi-copy groups of the polypeptide compounds in the same group are the same, only the quantities of branches are different. The two groups of compounds are selected randomly from the embodiments. The detection results of the embodiments 2-1, 2-14, 4-1, 4-14, 8-1 and 8-14 are shown in Table 6. The results of the other embodiments have little difference from those shown in Table 5, and are omitted here.

Group division:
Blank group: 0.3ml normal saline is injected;
Reference group: 0.3ml live NDV vaccine (abbreviated as "vaccine", CS2 strain) is inoculated;
Experimental group: 0.3ml vaccine mixed with 0.2µg polypeptide compounds in the embodiments is inoculated;
The dietetic activities of the SPF chicks in the groups are normal during the experiment, no adverse reaction is seen, and no SPF chick dies. That indicates the polypeptide compound provided in the present invention is safe to use.

**Table 6. Result of Immunity Experiment of SPF Chicks**

| Group | | Inoculated Substance | | Average Antibody Titer |
|---|---|---|---|---|
| | | Vaccine | Embodiment | |
| Blank group | | No | No | Negative |
| Reference group | | Vaccine | No | 8.2log4 |
| Experimental group | Group I | Vaccine | 2-1 | 9.2log5 |
| | | Vaccine | 4-1 | 9.6log4 |
| | | Vaccine | 8-1 | 10.6log3 |
| | Group II | Vaccine | 2-14 | 9.5log3 |
| | | Vaccine | 4-14 | 9.9log5 |
| | | Vaccine | 8-14 | 10.7log3 |

| | | | | |
|---|---|---|---|---|
| Note: "Negative" refers to that the HI antibody titer is zero. | | | | |

The results in Table 6 indicates that the immune response effect to the chicks is improved and the immunity enhancement effect is further improved as the number of copies of the polypeptide segment is increased (i.e., the quantity of branches is increased). That means the biological effect is positively correlated to the number of copies of the peptide fragments.

### Industrial Applicability

The polypeptide compound provided in the present invention is hopeful to become the effective ingredient in a variety of medicines, and is applicable to medicines for preventing and treating many diseases. Especially, the polypeptide compound can be used to prepare medicines for enhancing immune ability, and is suitable for industrial application.

## Claims

1. A polypeptide compound, having a structural formula selected from (X_{A}X_{B}X_{C}X_{D}X_{E}X_{F}X_{G}-X)₂KY or {(X_{A}X_{B}X_{C}X_{D}X_{E}X_{F}X_{G}-X)₂K}₂KY or {({X_{A}X_{B}X_{C}X_{D}X_{E}X_{F}X_{G}-X}₂K)₂K}₂KY;
wherein X_{A}, X_{B}, X_{D}, X_{E} and X_{G} are one of aliphatic amino acid molecules respectively, X_{C} and X_{F} are aliphatic amino acid molecules or heterocyclic amino acid molecules, K is lysine (Lys, K), X and Y are null or any one or more amino acid or chemical groups.

2. The polypeptide compound according to claim 1, wherein, the (X_{A}X_{B}X_{C}X_{D}X_{E}X_{F}X_{G}-X)₂KY structure is shown in formula 4:

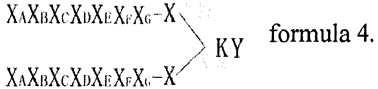

3. The polypeptide compound according to claim 1, wherein, the {(X_{A}X_{B}X_{C}X_{D}X_{E}X_{F}X_{G}-X)₂K}₂KY structure is shown in formula 5:

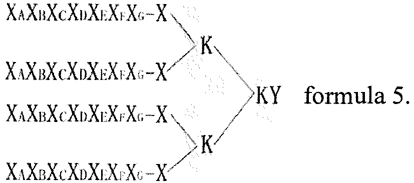

4. The polypeptide compound according to claim 1, wherein, the {({X_{A}X_{B}X_{C}X_{D}X_{E}X_{F}X_{G}-X}₂K) ₂K}₂KY structure is shown in formula 6:

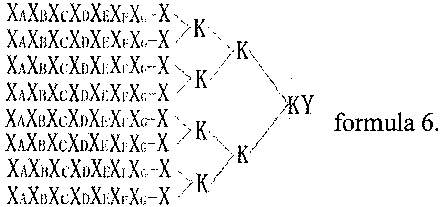

5. The polypeptide compound according to any of claim 1-4, wherein, X and Y are null, or any amino acid, or peptide fragments composed of any number of amino acids, or chemical groups that can connect amino acids or peptide fragments, and X and Y may be the same or different from each other; or X is null and Y is glycine (Gly, G).

6. The polypeptide compound according to any of claim 1-5, wherein, X_{A}, X_{B}, X_{D}, X_{E} and X_{G} are selected from alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E) or glutamine (Gln, Q) respectively, and X_{A}, X_{B}, X_{D}, X_{E} and X_{G} may be the same of different; or X_{A} is glycine (Gly, G), threonine (Thr, T), arginine (Arg, R), glutamate (Glu, E), alanine (Ala, A), lysine (Lys, K), leucine (Leu, L) or serine (Ser, S), or X_{A} is glycine (Gly, G), threonine (Thr, T) or arginine (Arg, R); or X_{B} is glutamine (Gln, Q), glutamate (Glu, E), arginine (Arg, R), alanine (Ala, A), threonine (Thr, T), leucine (Leu, L) or lysine (Lys, K), or X_{B} is glutamine (Gln, Q), arginine (Arg, R) or lysine (Lys, K); or X_{D} is arginine (Arg, R), serine (Ser, S), leucine (Leu, L), lysine (Lys, K), aspartate (Asp, D), glycine (Gly, G) or glutamate (Glu, E), or X_{D} is arginine (Arg, R), lysine (Lys, K) or glycine (Gly, G); or X_{E} is arginine (Arg, R), lysine (Lys, K), leucine (Leu, L) or glutamine (Gln, Q), or X_{E} is arginine (Arg, R) or lysine (Lys, K); or X_{G} is arginine (Arg, R), glycine (Gly, G), valine (Val, V), lysine (Lys, K), leucine (Leu, L) or glutamate (Glu, E), or X_{G} is arginine (Arg, R), glycine (Gly, G), valine (Val, V) or glutamate (Glu, E).

7. The polypeptide compound according to any of claims 1-6, wherein, X_{C} and X_{F} are selected from alanine (Ala, A), valine (Val, V), leucine (Leu, L), isoleucine (Ile, I), methionine (Met, M), cysteine (Cys, C), arginine (Arg, R), lysine (Lys, K), glycine (Gly, G), serine (Ser, S), threonine (Thr, T), aspartate (Asp, D), asparagine (Asn, N), glutamate (Glu, E), glutamine (Gln, Q), tryptophan (Trp, W), histidine (His, H) or proline (Pro, P) respectively, and X_{C} and X_{F} may be the same or different; or X_{C} is lysine (Lys, K), proline (Pro, P), tryptophan (Trp, W), alanine (Ala, A), leucine (Leu, L), histidine (His, H) or aspartate (Asp, D), or X_{C} is proline (Pro, P), leucine (Leu, L) or histidine (His, H); or X_{F} is proline (Pro, P), glutamate (Glu, E), aspartate (Asp, D), histidine (His, H), glycine (Gly, G), alanine (Ala, A) or lysine (Lys, K), or X_{F} is proline (Pro, P), glutamate (Glu, E), aspartate (Asp, D), histidine (His, H) or lysine (Lys, K).

8. The polypeptide compound according to any of claims 1-7, wherein, X is tyrosine (Tyr, Y), arginine (Arg, R), serine (Ser, S), asparagine (Asn, N), glycine (Gly, G), glutamate (Glu, E) or null, or X is tyrosine (Tyr, Y), arginine (Arg, R) or null; Y is glycine (Gly, G), alanine (Ala, A), cysteine (Cys, C) or null, or Y is glycine (Gly, G), alanine (Ala, A) or cysteine (Cys, C).

9. The polypeptide compound according to any of claims 1-8, further comprising a salt compound formed by the polypeptide compound with an organic acid or inorganic acid.

10. The polypeptide compound according to any of claims 1-8, further comprising an ether, ester, glucoside, or glycoside compound, ormed by a hydroxyl group included in the polypeptide compound.

11. The polypeptide compound according to any of claims 1-8, further comprising a thioether or thioglycoside compound, formed by a sulfhydryl group included in the polypeptide compound, or further comprising a compound containing disulfide bonds, which may be formed by sulfhydryl groups included in the polypeptide compound with cysteine or a peptide containing cysteine.

12. The polypeptide compound according to any of claims 1-8, further comprising an acylate or alkylate compound, formed by an amino group included in the polypeptide compound, or further comprising a glucoside compound formed by an amino group included in the polypeptide compound with saccharides.

13. The polypeptide compound according to any of claims 1-8, further comprising an ester or amide compound formed by a carboxyl group included in the polypeptide compound.

14. The polypeptide compound according to any of claims 1-8, further comprising a glucoside, acylate, or alkylate compound formed by an imino group included in the polypeptide compound.

15. The polypeptide compound according to any of claims 1-8, further comprising an ester, ether, glucoside, or glycoside compound formed by a phenolic hydroxyl included in the polypeptide compound, or a salt compound, formed by a phenolic hydroxyl included in the polypeptide compound with organic alkalis or inorganic alkalis.

16. The polypeptide compound according to any of claims 1-8, further comprising a coordinate, clathrate, or chelate compound formed by the polypeptide compound with metal ions.

17. The polypeptide compound according to any of claims 1-8, further comprising a hydrate or solvent formed by the polypeptide compound.

18. A pharmaceutical composition comprising the polypeptide compound according to any of claims 1-17, or a geometrical isomer of the polypeptide compound, a pharmaceutically acceptable salt or solvated compound of the polypeptid compound, and a pharmaceutical carrier or excipient.

19. A method for preparing the polypeptide compound according to any of claims 1-17, wherein, a synthesis route of (X_{A}X_{B}X_{C}X_{D}X_{E}X_{F}X_{G}-X)₂KY is expressed by formula 1: 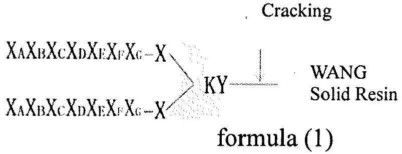
Y is first fixed to a WANG solid resin, and then is bonded with lysine Fmoc-Lys(Fmoc)-OH(Lys, K) by condensation, to form a two-branch skeleton ">KY-WANG solid resin" with branch nodes;
terminal amino groups of K in the ">KY-WANG solid resin" are bonded with a X_{A}X_{B}X_{C}X_{D}X_{E}X_{F}X_{G}-X segment respectively, to form ta wo-branch peptide (X_{A}X_{B}X_{C}X_{D}X_{E}X_{F}X_{G}-X)₂KY-WANG solid resin; or the two active terminal amino groups of K in the ">KY-WANG solid resin" are bonded with amino acids X, X_{G}, X_{F}, X_{E}, X_{D}, X_{C}, X_{B}, X_{A} by condensation in sequence, to obtain a (X_{A}X_{B}X_{C}X_{D}X_{E}X_{F}X_{G}-X)₂KY-WANG solid resin;
finally, the two-branch peptide is cracked from the WANG solid resin and then purified, to obtain a polypeptide compound (X_{A}X_{B}X_{C}X_{D}X_{E}X_{F}X_{G}-X)₂KY with two copies of X_{A}X_{B}X_{C}X_{D}X_{E}X_{F}X_{G}-X.

20. A method for preparing the polypeptide compound according to any of claims 1-17, wherein, a synthesis route of the {(X_{A}X_{B}X_{C}X_{D}X_{E}X_{F}X_{G}-X)₂K}₂KY structure is expressed by formula 2: 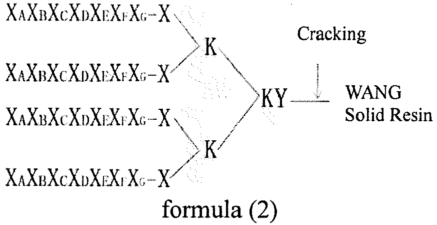
Y is first fixed to WANG solid resin first, and then is bonded with lysine Fmoc-Lys(Fmoc)-OH by condensation, to form a two-branch skeleton ">KY-WANG solid resin" with branch nodes; then, the two active terminal amino groups of K in the ">KY-WANG solid resin" are bonded with the terminal carboxyl groups of lysine Fmoc-Lys(Fmoc)-OH by condensation, to form a four-branch skeleton ">K₂KY-WANG solid resin" with two branch nodes;
next, the two active terminal amino groups of each lysine K in the ">K₂KY-WANG solid resin" are bonded with a X_{A}X_{B}X_{C}X_{D}X_{E}X_{F}X_{G}-X segment respectively, to form a four-branch peptide (X_{A}X_{B}X_{C}X_{D}X_{E}X_{F}X_{G}-X)₂KY-WANG solid resin; or the two active terminal amino groups of K in the ">K₂KY-WANG solid resin" are bonded with amino acids X, X_{G}, X_{F}, X_{E}, X_{D}, X_{C}, X_{B}, X_{A} by condensation in sequence, to obtain a (X_{A}X_{B}X_{C}X_{D}X_{E}X_{F}X_{G}-X)₄K₂KY-WANG solid resin;
finally, the four-branch peptide is cracked from the WANG solid resin and is purified, to obtain a polypeptide compound {(X_{A}X_{B}X_{C}X_{D}X_{E}X_{F}X_{G}-X)₂K}₂KY with four copies of X_{A}X_{B}X_{C}X_{D}X_{E}X_{F}X_{G}-X.

21. A method for preparing the polypeptide compound according to any of claims 1-17, wherein, a synthesis route of the {({X_{A}X_{B}X_{C}X_{D}X_{E}X_{F}X_{G}-X}₂K)₂K}₂KY structure is expressed by formula 3: 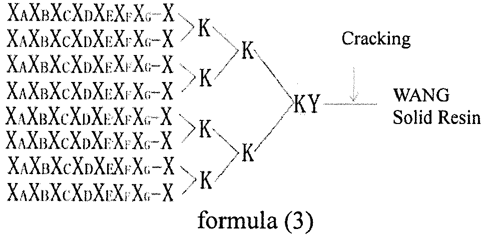
Y is fixed to WANG solid resin first, and then is bonded with lysine Fmoc-Lys(Fmoc)-OH by condensation, to form a two-branch skeleton ">KY-WANG solid resin" with branch nodes; the two active terminal amino groups of K are bonded with the carboxyl terminals of lysine Fmoc-Lys(Fmoc)-OH by condensation, to form a four-branch skeleton "K₂KY-WANG solid resin"; then, the two active terminal amino groups of K in the four-branch skeleton ">K₂KY-WANG solid resin" are bonded with the terminal carboxyl groups of lysine Fmoc-Lys(Fmoc)-OH by condensation, to form an eight-branch skeleton ">K₄K₂KY-WANG solid resin" with four branch nodes;
next, the two active terminal amino groups of each lysine K in the ">K₄K₂KY-WANG solid resin" are bonded with a X_{A}X_{B}X_{C}X_{D}X_{E}X_{E}X_{G}-X segment respectively, to form eight-branch peptide (X_{A}X_{B}X_{C}X_{D}X_{E}X_{F}X_{G}-X)₈K₄KY-WANG solid resin; or the two active terminal amino groups of K in the ">K₄K₂KY-WANG solid resin" are bonded with amino acids X, X_{G}, X_{F}, X_{E}, X_{D}, X_{C}, X_{B}, X_{A} by condensation in sequence, to obtain (X_{A}X_{B}X_{C}X_{D}X_{E}X_{F}X_{G}-X)₈K₄K₂KY-WANG solid resin;
finally, the eight-branch peptide is cracked from the WANG solid resin and purified, to obtain a polypeptide compound {({X_{A}X_{B}X_{C}X_{D}X_{E}X_{F}X_{G}-X}₂K)₂K}₂KY with eight copies of X_{A}X_{B}X_{C}X_{D}X_{E}X_{F}X_{G}-X.

22. The method according to any of claims 19-21, wherein, before the terminal carboxyl group of K is condensed with the Y-WANG solid resin, the two amido groups of K are protected with thet-butyloxycarbonyl (Boc) protection group/method or with the fluorenylmethoxycarbonyl (Fmoc) protection group/method.

23. The method according to any of claims 19-21, wherein, before the terminal carboxyl groups of the other two lysines are condensed with the two terminal amino groups of K in KY, the two amino groups of each lysine are protected; before the carboxyl terminal of the X_{A}X_{B}X_{C}X_{D}X_{E}X_{F}X_{G}-X is condensed with the amino terminal of each lysine, the amido group of the X_{A}X_{B}X_{C}X_{D}X_{E}X_{F}X_{G}-X is protected with thet-butyloxycarbonyl (Boc) protection group/method or with the fluorenylmethoxycarbonyl (Fmoc) protection group/method.

24. The method according to any of claims 19-23, comprising the following steps:
step 1: protecting the two amino groups of the lysine K with an Fmoc protection group/method;
step 2: fixing KY to the WANG solid resin with an automatic polypeptide synthesizer, in the following bonding sequence: KY-WANG solid resin;
when the two-copy polypeptide compound is prepared, the two activated terminal amino groups of lysine in KY are further condensed with another two X_{A}X_{B}X_{C}X_{D}X_{E}X_{F}X_{G}-X fragments, to obtain the polypeptide compound (X_{A}X_{B}X_{C}X_{D}X_{E}X_{F}X_{G}-X)₂KY with two copies of X_{A}X_{B}X_{C}X_{D}X_{E}X_{F}X_{G}-X, which is fixed to the WANG solid resin; or the two activated terminal amino groups of the lysine in KY are further condensed with another two lysines K, in each of which the two amino groups have been protected with an Fmoc protection method, to obtain a two-branch skeleton K₂KY-WANG solid resin;
when the four-copy polypeptide compound is prepared, the two activated terminal amino groups of each lysine in the two-branch skeleton "K₂" are further condensed with two X_{A}X_{B}X_{C}X_{D}X_{E}X_{F}X_{G}-X fragments, to obtain the polypeptide compound {(X_{A}X_{B}X_{C}X_{D}X_{E}X_{F}X_{G}-X)₂K}₂KY with four copies of X_{A}X_{B}X_{C}X_{D}X_{E}X_{F}X_{G}-X, which is fixed to the WANG solid resin; or the two activated terminal amino groups of each lysine in the two-branch skeleton "K₂" are further condensed with another two lysines K, in each of which the two amido groups have been protected with an Fmoc protection method, to obtain a four-branch skeleton K₄K₂KY-WANG solid resin; or
when the eight-copy polypeptide compound is prepared, the two activated terminal amino groups of each lysine in the four-branch skeleton "K₄" are further condensed with two X_{A}X_{B}X_{C}X_{D}X_{E}X_{F}X_{G}-X fragments, to obtain the polypeptide compound {({X_{A}X_{B}X_{C}X_{D}X_{E}X_{F}X_{G}-X}₂K)₂K}₂KY with eight copies of X_{A}X_{B}X_{C}X_{D}X_{E}X_{F}X_{G}-X, which is fixed to the WANG solid resin;
finallywhere, the polypeptide compound is cleaved from the WANG solid resin with a TFA method, to obtain a crude polypeptide compound product;
step 3: purifying the crude polypeptide compound product with a chromatographic column (model: Daiso C18, 10µm, 100Å, 50x250mm), wherein, the mobile phase A is an aqueous solution that contains 0.05% trifluoroacetic acid and 2% acetonitrile, the mobile phase B is 90% acetonitrile/water, the flow rate is 25mL/min., and the ultraviolet detection wavelength is 220nm; the eluting peak solution is collected and then freeze-dried, to obtain a white flocculent polypeptide compound.

25. An application of the polypeptide compound according to any of claims 1-17 in preparation of immunize medicines for humans or animals or medicines for enhancing the immune function of humans or animals.

26. An application of a polypeptide compound prepared with the method according to any of claims 19-24 in preparation of immunize medicines for humans or animals or medicines for enhancing the immune function of humans or animals.

27. An application of the polypeptide compound according to any of claims 1-17 or a polypeptide compound prepared with the method according to any of claims 19-24 in preparation of medicines for inhibiting tumor growth in humans or animals.

28. The application according to claim 27, wherein, the tumor is a solid tumor, a residual tumor after medical operation, or a hematological tumor, wherein the hematological tumor is selected from leukaemia and lymphoma in a human body.

29. The Application according to claim 27 or 28, wherein, the tumor is selected fromsarcoma, liver cancer, colon cancer, lung cancer, stomach cancer, mammary cancer, and cervical cancer.

30. An application of the polypeptide compound according to any of claims 1-17 or a polypeptide compound prepared with the method according to any of claims 19-24 in preparation of anti-infection or anti-virus medicine for humans or animals.

31. An application of the polypeptide compound according to any of claims 1-17 or a polypeptide compound prepared with the method according to any of claims 19-24 in molecular tracers.

32. An application of the polypeptide compound according to any of claims 1-17 or a polypeptide compound prepared with the method according to any of claims 19-24 in preparation of medicine for treating diseases of humans incurred by vascular proliferation (including, but not limited to application of medicines for treating maculopathy in fundus).
